# EUROPÄISCHE PATENTSCHRIFT

(11) **EP 1 746 956 B1**
(45) Veröffentlichungstag und Bekanntmachung des Hinweises auf die Patenterteilung: **03.10.2007**
(21) Anmeldenummer: 05736345.9
(22) Anmeldetag: 27.04.2005
(51) Int. Cl.: A61F 2/64, A61F 2/68

(54) **SCHWUNGPHASENSTEUERVORRICHTUNG**
SWING PHASE CONTROL DEVICE
DISPOSITIF DE COMMANDE DE PHASE CENTRIFUGE

(30) Priorität: 18.05.2004 DE 202004008014 U
(43) Veröffentlichungstag der Anmeldung: 31.01.2007
(73) Patentinhaber: TEKO Automation, Mensch und Technik GmbH, 78054 VS-Schwenningen (DE)
(72) Erfinder: BISINGER, Hardy, 72348 Rosenfeld (DE); MAREK, Volker, 78056 VS-Schwenningen (DE); FITZLAFF, Gerhard, 78054 VS-Schwenningen (DE)
(74) Vertreter: Hofer, Dorothea
(86) Internationale Anmeldenummer: PCT/EP2005/004520
(87) Internationale Veröffentlichungsnummer: WO 2005/115279

(56) Entgegenhaltungen:
- EP-A- 1 101 461
- EP-B- 0 615 430
- DE-C1- 4 338 946
- DE-U1- 20 313 999
- GB-A- 2 282 414
- US-A- 2 568 051
- US-A- 5 383 939
- US-A- 5 904 721

## Beschreibung

Die vorliegende Erfindung bezieht sich auf eine Schwungphasensteuervorrichtung für ein künstliches Kniegelenk mit einer Kolbenzylindereinrichtung.

Eine solche Kolbenzylindereinrichtung ist z.B. aus der EP 0 615 430 B1 bekannt. Die dort beschriebene Schwungphasensteuervorrichtung weist eine erste Kammer auf der einen Seite des Kolbens auf und eine zweite Kammer auf der gegenüberliegenden Seite des Kolbens, wobei die beiden Kammern über eine erste Drossel miteinander verbindbar sind. Eine zweite Drossel ist zum Verbinden der zweiten Kammer mit einem Fluidreservoir vorgesehen, wobei eine Einrichtung so ausgebildet ist, dass sie bei Erreichen bzw. Überschreiten eines vorbestimmten Drucks in der zweiten Kammer die zweite Drossel schließt.

Mit dieser Schwungphasensteuerung wird erreicht, dass das Eintauchen des Kolbens der Kolbenzylindereinrichtung und damit das Beugen des künstlichen Kniegelenks durch die zweite Drossel gesteuert werden kann und die Drosselung abhängig von der Eintauchgeschwindigkeit des Kolbens erfolgt. Damit können unterschiedliche Steuerungen für das Beugen des Kniegelenks realisiert werden und somit wird ein natürlicherer Bewegungsablauf ermöglicht.

Wenn das Kniegelenk wieder gestreckt werden soll, muss die Person, die das künstliche Kniegelenk benutzt, den Unterschenkel wieder nach vorne schwingen und in die Streckung mit dem Oberschenkel bringen. Hierbei tritt bei der bekannten Schwungphasensteuerung eine vorbestimmte Drosselwirkung auf, bei der die Drosselung über die erste Drossel erfolgt und nicht von der Streckgeschwindigkeit des künstlichen Kniegelenks abhängt.

Da bei einem natürlichen Bewegungsablauf jedoch auch der Streckvorgang abhängig von der Schrittgeschwindigkeit erfolgt, führt eine solche Drosselung zu einem unnatürlichen Bewegungsablauf. Zwar ist die erste Drossel einstellbar und kann damit an den jeweiligen Laufstil angepasst werden, jedoch weist sie keine dynamische Anpassung an unterschiedliche Bewegungsabläufe der Person auf. Die erste Drossel wirkt also nur optimal entweder für langsame oder für schnelle Bewegungen.

Es ist Aufgabe der Erfindung, eine Schwungphasensteuerung der eingangs beschriebenen Art zu schaffen, die einen natürlichen Bewegungsablauf ermöglicht und die insbesondere auch für unterschiedlich schnelle Streckbewegungen des künstlichen Kniegelenks geeignet ist.

Die Aufgabe wird gelöst durch eine Schwungphasensteuerung gemäß Anspruch 1. Weiterbildungen der Erfindung sind in den Unteransprüchen gekennzeichnet.

Die erfindungsgemäße Schwungphasensteuerung weist den besonderen Vorteil auf, dass sie auch für die Streckbewegung eine Drosselung bereitstellt, die abhängig von der Bewegungsgeschwindigkeit ist und getrennt von der Drosselung der Beugebewegung einstellbar ist. Es wird somit ein Bewegungsablauf ermöglicht, der natürlicher wirkt. Wenn eine schnelle Streckbewegung erfolgt, verkleinert der sich aus dem Zylinder bewegende Kolben sehr schnell das Volumen der ersten Kammer und komprimiert das darin enthaltene Fluid. Überschreitet der Druck einen vorbestimmten Wert, wird die erste Drossel geschlossen und es tritt eine Dämpfung durch das komprimierte Fluid auf.

Mit der erfindungsgemäßen Schwungphasensteuerung ist folglich ein natürliches und schwungvolles Gehen mit der Prothese möglich.

Weitere Merkmale und Zweckmäßigkeiten der Erfindung ergeben sich aus der Beschreibung von Ausführungsbeispielen anhand der beigefügten Zeichnungen. Von den Figuren zeigen:
- Fig. 1: eine Seitenansicht einer Schwungphasensteuereinrichtung einer Ausführungsform der vorliegenden Erfindung;
- Fig. 2: eine Draufsicht auf die Schwungphasensteuereinrichtung aus Fig. 1;
- Fig. 3: einen Schnitt entlang der Linie A-A in Fig. 2;
- Fig. 4: einen Schnitt entlang der Linie B-B in Fig. 1;
- Fig. 5: eine Darstellung, die die Verwendung der Schwungphasensteuereinrichtung in einem künstlichen Knie einer Beinprothese zeigt;
- Fig. 6: eine schematische Darstellung einer Schwungphasensteuereinrichtung gemäß einer weiteren Ausführungsform der vorliegenden Erfindung bei einer Flexionsbewegung;
- Fig. 7: eine schematische Darstellung der in Fig. 6 gezeigten Schwungphasensteuereinrichtung bei einer Extensionsbewegung.

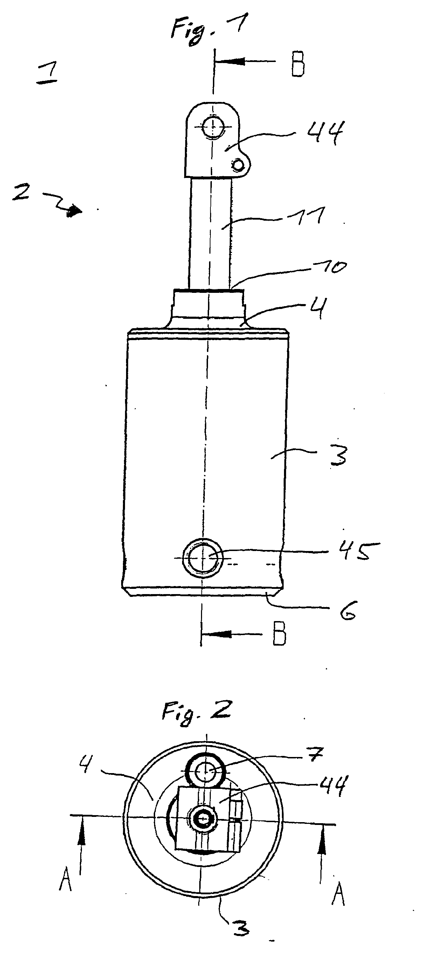

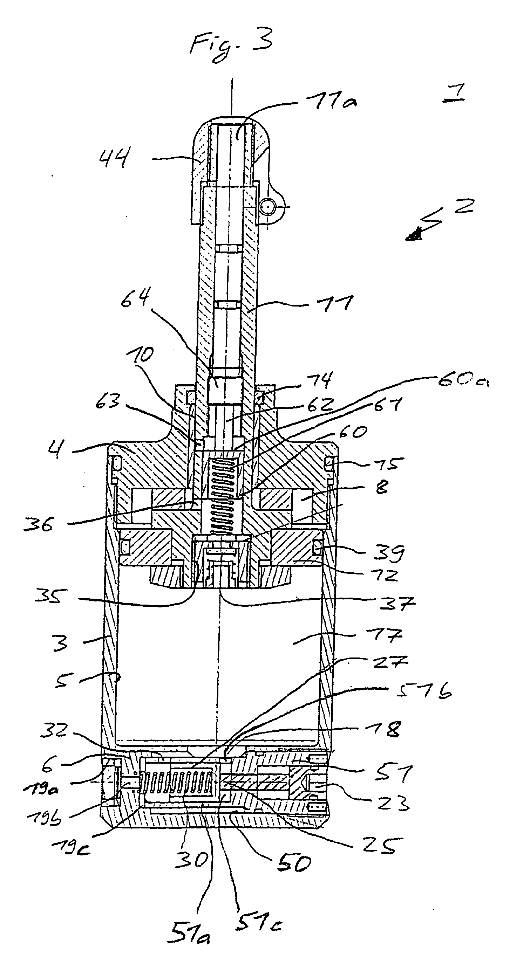

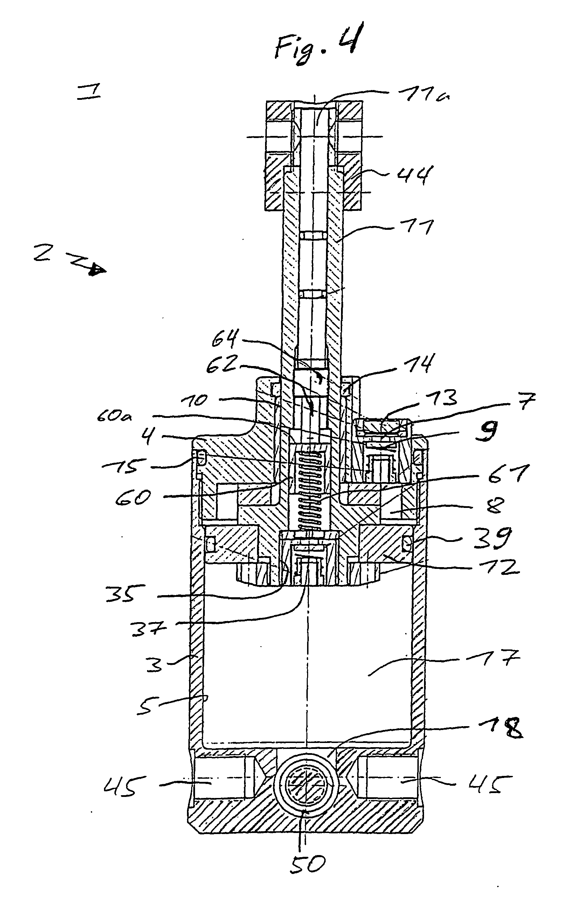

Im Folgenden wird mit Bezug auf die Fig. 1 bis 4 eine erste Ausführungsform der vorliegenden Erfindung beschrieben.

Die Schwungphasensteuervorrichtung 1 weist eine Zylinderkolbeneinrichtung 2 mit einem Zylinder 3 und einem Kolben 12 auf. Der Zylinder 3 umfasst einen rohrförmigen Abschnitt 5 mit einem Deckelteil 4 an seinem einen Ende und einem Bodenteil 6 an dem gegenüberliegenden Ende.

Das Deckelteil 4 weist eine Einlassöffnung 7 auf, die eine Verbindung zwischen der Umgebungsluft und einer in dem Zylinder 3 gebildeten eingangsseitigen ersten Kammer 8 ermöglicht. Die Einlassöffnung 7 ist mit einem Rückschlagventil 9 versehen, das sich zu der ersten Kammer 8 hin öffnet und das beispielsweise als Flatterventil ausgebildet sein kann. Konzentrisch zu dem Zylinder 3 ist eine Bohrung 10 in einer Buchse in dem Deckelteil 4 vorgesehen. In der Bohrung 10 befindet sich ein Gleitlager für eine Kolbenstange 11, die mit dem Kolben 12 der Kolbenzylindereinrichtung 2 verbunden ist. Auf der Außenseite der Einlassöffnung 7 ist ein Filter 13 zum Schutz gegen das Eindringen unerwünschter Bestandteile aus der Außenluft und zum Dämpfen von Geräuschen durch das Hindurchströmen von Luft vorgesehen. Zum Abdichten des Raumes zwischen der konzentrischen Bohrung 10 und der Kolbenstange 11 ist eine Ringdichtung 14 vorgesehen.

Das Deckelteil 4 ist mit dem rohrförmigen Abschnitt 5 verbunden und die Verbindung zwischen dem rohrförmigen Abschnitt 5 und dem Deckelteil 4 ist beispielsweise durch eine Ringdichtung 15 abgedichtet. Das Bodenteil 6 verschließt den rohrförmigen Abschnitt 5 an seinem dem Deckelteil 4 entgegengesetzten Ende und ist einstückig mit dem rohrförmigen Abschnitt 5 ausgebildet. Das Bodenteil 6 kann auch separat ausgebildet sein und mit dem rohrförmigen Abschnitt 5 verschraubt oder anderweitig verbunden sein.

Auf der der ersten Kammer 8 abgewandten Seite des Kolbens 12 ist eine zweite Kammer 17 in dem rohrförmigen Abschnitt 5 gebildet. Auf der dem Kolben 12 entgegengesetzten Seite ist die zweite Kammer 17 von dem Bodenteil 6 begrenzt.

In dem Bodenteil 6 ist konzentrisch eine in die zweite Kammer 17 mündende Bohrung 18 vorgesehen. Das Bodenteil 6 weist eine radiale Bohrung 50 auf, die die Außenseite des Zylinders 3, mit der Bohrung 18 verbindet und mit einem Verschlusselement 51 versehen ist. Die Bohrung 50 zu der entgegengesetzten Außenseite des Zylinders 3 fortsetzend ist eine radiale Auslassbohrung 19 vorgesehen, die die Außenseite des Zylinders 3 und damit die Umgebungsluft mit der Bohrung 18 verbindet. Der der Außenseite zugewandte Abschnitt 19a der Auslassbohrung 19 ist mit einem Filter 20 zum Verhindern des Eintretens von Staub aus der Umgebungsluft und zum Dämpfen von Geräuschen durch das Hindurchströmen von Luft durch die Auslassbohrung 19 versehen. Die radiale Auslassbohrung 19 weist in dem an den Abschnitt 19a in Richtung der Bohrung 18 angrenzenden Abschnitt 19b einen verjüngten Querschnitt auf und in dem direkt an die Bohrung 50 angrenzenden Abschnitt 19c wieder einen größeren Querschnitt, der etwas kleiner ist als der Querschnitt der Bohrung 50.

Der Durchgang von der Bohrung 18 zu dem Abschnitt 19c ist teilweise von dem Verschlusselement 51 verschlossen, so dass eine Drossel 32 gebildet ist, die ein Ausströmen der Luft aus der zweiten Kammer 17 über die Bohrungen 18, 50 und 19 drosselt. Das Verschlusselement 51 ist derart ausgebildet, dass es den der Bohrung 19 abgewandten Bereich der Bohrung 50 verschließt und mit einem hohlzylinderförmigen Fortsatz 51a in den Durchgang von der Bohrung 18 zu dem Abschnitt 19c ragt.

In dem hohlzylindrischen Innenraum des Verschlusselements 51 ist ein Steuerelement 27 gehalten, das in dem Verschlusselement 51 koaxial verschiebbar ist. Das Steuerelement 27 ist becherförmig ausgebildet und die Bodenfläche 27a des Bechers bildet eine Seitenwand eines Raums 51c in dem hohlzylindrischen Innenraum des Verschlusselements 51. Dieser Raum 51c steht über eine seitliche Bohrung 51b in dem hohlzylindrischen Fortsatz 51a mit der Bohrung 18 in Kontakt. In einer ersten Endstellung verschließt dieses Steuerelement 27 die Drossel 32 vollständig und in Richtung einer zweiten Endstellung gibt es die Drossel 32 sukzessiv frei. Dieses Steuerelement 27 wird von einer Druckfeder 30, die in den Innenraum des becherförmigen Steuerelements 27 eingreift, in die zweite Endstellung gegen einen Anschlag 25 vorgespannt, der in den Raum 51c ragt. Die Position des Anschlags 25 kann über eine von außen zugängliche Einstellschraube 23 eingestellt werden.

Wenn von der zweiten Kammer 17 Luft durch die Bohrung 18 in die Bohrung 50 strömt, drückt diese auf die der Druckfeder 30 abgewandten Seite gegen das Steuerelement 27 und bewegt dieses bei Überschreiten der Federkraft in Richtung der die Drossel 32 verschließenden Stellung.

Der Kolben 12 weist ferner eine konzentrische Bohrung 35 auf, die in die zweite Kammer 17 mündet. An einer der zweiten Kammer 17 abgewandten Seite steht die konzentrische Bohrung 35 über eine Querbohrung 36 mit der ersten Kammer in Verbindung. Die konzentrische Bohrung 35 weist ein sich zur zweiten Kammer hin öffnendes Rückschlagventil 37 auf, das wiederum beispielsweise als Flatterventil ausgebildet sein kann. Der Kolben 12 weist eine Ringdichtung 39 auf, die an den rohrförmigen Abschnitt 5 anliegt. Die konzentrische Bohrung 35 des Kolbens 12 setzt sich in der mit dem Kolben 12 an der dem Deckelteil 4 zugewandten Seite verbundenen Kolbenstange 11 fort. Die Kolbenstange 11 ist also in einem an die konzentrische Bohrung 35 anschließenden Abschnitt hohl mit einem zylindrischen Querschnitt ausgebildet.

In der Kolbenstange 11 ist ein koaxial zu dieser angeordnetes zweites Steuerelement 60 angeordnet. Das zweite Steuerelement 60 weist einen Außendurchmesser auf, der derart dem Innendurchmesser der hohlen Kolbenstange 11 entsprechend gewählt ist, dass das zweite Steuerelement 60 den Querschnitt der Kolbenstange 11 ausfüllt und in dieser entlang der Stangenachse verschiebbar ist. Das zweite Steuerelement 60 ist in der Kolbenstange 11 derart angeordnet, dass es in einer ersten Endstellung die Querbohrung 36 vollständig verschließt, die eine Drossel für Luft bildet, die von der ersten Kammer 8 in Richtung der zweiten Kammer 17 strömt, und bei Bewegung in Richtung einer zweiten Endstellung die Querbohrung 36 sukzessiv freigibt.

Auf der dem Kolben 12 zugewandten Seite des zweiten Steuerelements 60 ist in der Kolbenstange 11 eine zweite Druckfeder 61 vorgesehen, die das zweite Steuerelement 60 in Richtung der zweiten Endstellung gegen einen in der Kolbenstange 11 vorgesehenen Anschlag 62 vorspannt.

In der Kolbenstange 11 ist auf der dem Kolben 12 abgewandten Seite des zweiten Steuerelements 60 eine zweite Querbohrung 63 vorgesehen, die den Innenraum der hohlen Kolbenstange 11 mit der ersten Kammer 8 verbindet. In Richtung des freien Endes 11a der Kolbenstange 11 ist der Innenraum der hohlen Kolbenstange 11 durch ein zweites Verschlusselement 64 begrenzt. Über die zweite Querbohrung 63 wirkt der Druck in der ersten Kammer 8 auf die der zweiten Druckfeder 61 abgewandte Oberseite 60a des zweiten Steuerelements 60. Über eine vom freien Ende 11a zugängliche Schraube kann das zweite Verschlusselement 64 in seiner Position in Richtung der Kolbenstangenachse verstellt werden, sodass mit dem zweiten Steuerelement 60 über den Anschlag 62 die Querbohrung 36 entgegen dem Druck der zweiten Druckfeder 61 teilweise vorverschlossen werden kann. Mit dieser Anordnung ist eine einstellbare Drossel ausgebildet, deren Querschnitt abhängig von dem Druck in der ersten Kammer und damit von der Ausfahrgeschwindigkeit des Kolbens aus dem Zylinder verjüngt wird.

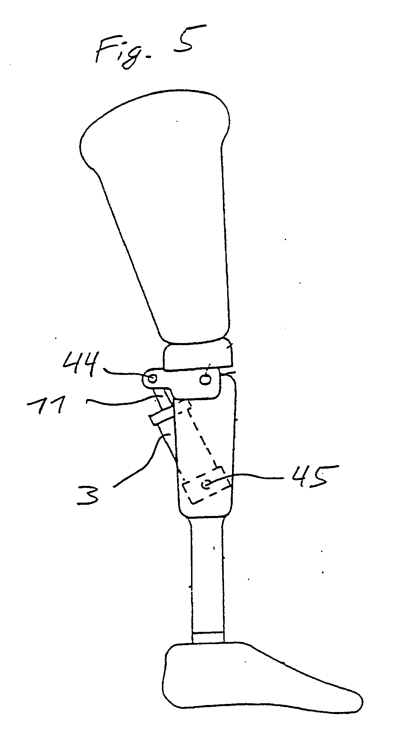

Bei der Benutzung in einem künstlichen Kniegelenk, die in Fig. 5 dargestellt ist, wird die Schwungphasensteuervorrichtung 1 mit einem mit dem freien Ende 11a der Kolbenstange 11 verbundenen Befestigungsteil 44 mit dem Prothesenoberschenkelteil verbunden. Der Zylinder 3 wird in seinem Bodenbereich durch darin ausgebildete Ausnehmungen 45 mit dem Prothesenunterteil verbunden.

Im Folgenden wird der Betrieb der Schwungphasensteuerungsvorrichtung beschrieben. Wenn das künstliche Kniegelenk gestreckt ist (Extension), befindet sich der Kolben 12 in seiner zurückgezogenen oberen, dem Deckelteil 4 benachbarten Stellung. Die erste Kammer 8 hat ihre minimale Größe. Diese Situation ist in den Fig. 1, 3 und 4 jeweils dargestellt. Die zweite Kammer 17 hat ihre maximale Größe. Wenn das künstliche Bein der Person, die die Schwungphasensteuervorrichtung verwendet, gebeugt wird, knickt das Kniegelenk ein (Flexion). Der Kolben 12 bewegt sich von seiner oberen Stellung in die untere Stellung und Umgebungsluft strömt durch das Rückschlagventil 9 in die sich vergrößernde erste Kammer 8 nach. Die Druckfeder 30 und der Durchmesser der Drossel 32 sind so bemessen, dass bei normaler Betätigung der Druck in der zweiten Kammer 17 das Steuerelement 27 noch nicht in eine die Drossel 32 verschließende Stellung drücken kann. Daher ist die Drossel 32 frei und der nach unten gehende Kolben 12 verdrängt die in der zweiten Kammer 17 vorhandene Luft durch die Bohrungen 18, 50 und 19 nach außen.

Wenn sich dagegen die die Prothese tragende Person schnell bewegt und dabei das Kniegelenk beugt, bewegt sich der Kolben 12 schnell in die untere Position. Dabei baut sich in der zweiten Kammer 17 schnell ein hoher Druck auf, da die Luft durch die Drossel 32 nicht schnell genug von der zweiten Kammer 17 über die Bohrung 18 und die Drossel 32 zu der Auslassbohrung 19 abfließen kann. Falls die Beugebewegung lange genug anhält, steigt der Druck in der zweiten Kammer 17 so hoch an, dass der auf das Steuerelement 27 wirkende Druck ausreicht, um das Steuerelement 27 gegen die Wirkung der Druckfeder 30 in eine die Drossel 32 etwas verschließende Stellung zu bewegen. Durch das Verschließen der Drossel 32 steigt der Druck in der zweiten Kammer 17 noch rascher an und das Steuerelement 27 verschließt die Drossel 32 noch schneller, sodass sie schließlich ganz verschlossen ist. Dann wirkt die zweite Kammer als Luftfeder, da die Luft nur noch komprimiert wird, und nicht mehr abfließen kann.

Die durch die zweite Kammer 17 gebildete Luftfeder bewirkt, dass die eintauchende Bewegung des Kolbens 12 in dem Zylinder 3 umgekehrt wird und der Kolben 12 wieder aus dem Zylinder 3 herausgeschoben wird. Da die Aufwärtsbewegung des Kolbens 12 eine Streckung des Kniegelenks bedeutet, wird also die Streckbewegung des Kniegelenks durch das Umkehren der Bewegung des Kolbens 12 das durch die Luftfeder in der zweiten Kammer 17 verursacht wird, aktiv unterstützt.

Bei der Streckbewegung des Kniegelenks, wenn der Kolben 12 wieder aus dem Zylinder 3 herausgeschoben wird, strömt Luft aus der ersten Kammer 8 durch die Querbohrung 36, die Bohrung 35 und das in dieser Richtung durchlässige Rückschlagventil 37 in die sich vergrößernde zweite Kammer 17. Die zweite Druckfeder 61 und der Durchmesser der Querbohrung 36 sind so bemessen, dass bei normaler Betätigung der Druck in der ersten Kammer 8 das zweite Steuerelement 60 noch nicht in eine die Querbohrung 36 verschließende Stellung drücken kann. Daher ist die Querbohrung 36 frei und der nach oben gehende Kolben 12 verdrängt die in der ersten Kammer 8 vorhandene Luft in die zweite Kammer 17.

Wenn sich dagegen die die Prothese tragende Person wiederum schnell bewegt und dabei das Kniegelenk streckt, bewegt sich der Kolben 12 schnell in die obere Position. Dabei baut sich in der ersten Kammer 8 schnell ein hoher Druck auf, da die Luft durch die Querbohrung 36 nicht schnell genug über die Querbohrung 36 und die Bohrung 35 in die zweite Kammer 17 abfließen kann. Falls die Streckbewegung lange genug anhält, steigt der Druck in der ersten Kammer 8 so hoch an, dass der auf das zweite Steuerelement 60 wirkende Druck ausreicht, um das zweite Steuerelement 60 gegen die Wirkung der zweiten Druckfeder 61 in eine die Querbohrung 36 etwas verschließende Stellung zu bewegen. Durch das Verschließen der Querbohrung 36 steigt der Druck in der ersten Kammer 8 noch rascher an und das zweite Steuerelement 60 verschließt die Querbohrung 36 noch schneller, sodass sie schließlich ganz verschlossen ist. Dann wirkt die erste Kammer als Luftfeder.

Die durch die erste Kammer 8 gebildete Luftfeder bewirkt, dass die herausschiebende Bewegung des Kolbens 12 aus dem Zylinder 3 umgekehrt wird und der Kolben 12 wieder in den Zylinder 3 eingetaucht wird. Da die Abwärtsbewegung des Kolbens 12 eine Beugung des Kniegelenks bedeutet, wird also die Beugebewegung des Kniegelenks durch das Umkehren der Bewegung des Kolbens 12, das durch die Luftfeder in der ersten Kammer 8 verursacht wird, ebenfalls aktiv unterstützt.

Also werden die Beuge- und die Streckbewegung des Kniegelenks abhängig von der Bewegungsgeschwindigkeit aktiv unterstützt, sodass sich die Person viel schneller und sicherer bewegen kann. Die Zeitpunkte der jeweiligen durch die Luftfedern bedingten Umkehrbewegungen können durch Einstellen der jeweiligen Drosselungen durch Verstellen der Anschläge 25, 62 an die individuellen Gegebenheiten angepasst werden.

Bei der oben beschriebenen Ausführungsform sind die erste Kammer 8 und die zweite Kammer 17 über die Einlassöffnung 7 bzw. die Auslassbohrung 19 mit der Umgebungsluft verbunden. Es handelt sich also um ein offenes System, bei dem als Medium Luft verwendet wird. Es kann aber auch ein geschlossenes System vorgesehen sein, bei dem die Schwungphasensteuerung von einem hermetisch abgeschlossenen Außengehäuse umgeben ist. Im Fall einer solchen alternativen Ausführungsform kann auch ein anderes Gas oder eine Flüssigkeit als bewegtes Medium verwendet werden. Mit einer solchen Ausführungsform kann die Kennlinie der Schwungphasensteuerung beeinflusst werden. Die Verwendung einer Flüssigkeit weist aber aufgrund der geringen Kompressibilität nicht die rückfedernde Eigenschaft auf, die zuvor beschrieben wurde.

Es ist in einer weiteren Ausführungsform ebenfalls möglich, die in dem Kolben 12 und der Kolbenstange 11 vorgesehene Drosseleinrichtung 36, 63, 60, 61 umzudrehen, sodass sie als Drosseleinrichtung für ein aus der zweiten Kammer 17 in die erste Kammer 8 strömendes Fluid dient, und die andere Drosseleinrichtung 18, 19, 32, 30, 27 in der ersten Kammer 8 zwischen der ersten Kammer und der Außenseite des Zylinders 3 vorzusehen, sodass sie als Drosseleinrichtung für ein von der ersten Kammer 8 zu der Außenseite strömendes Fluid dient. Die Einlassöffnung 7 mit Rückschlagventil 9 ist in diesem Fall in der zweiten Kammer 17 angeordnet.

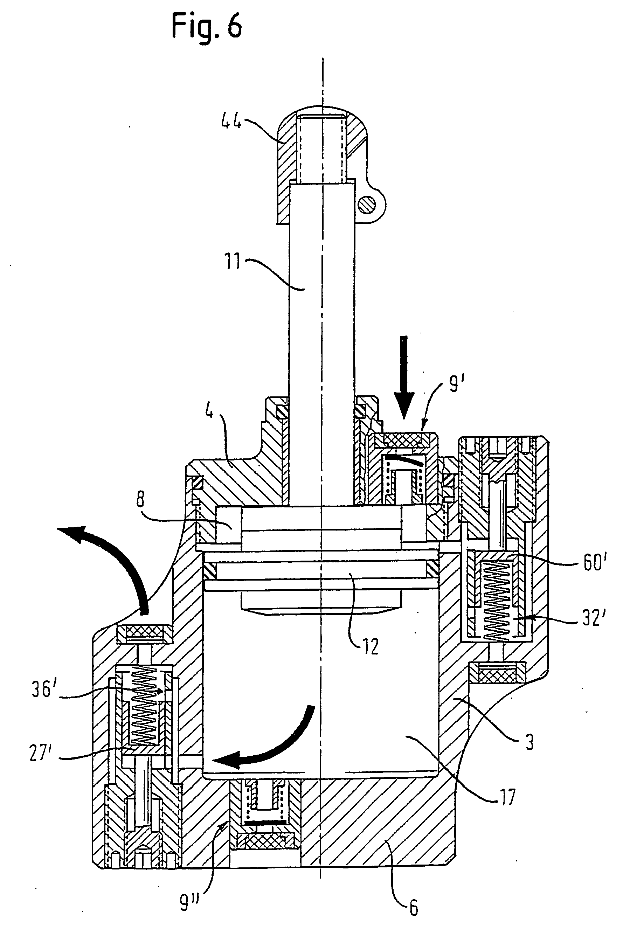

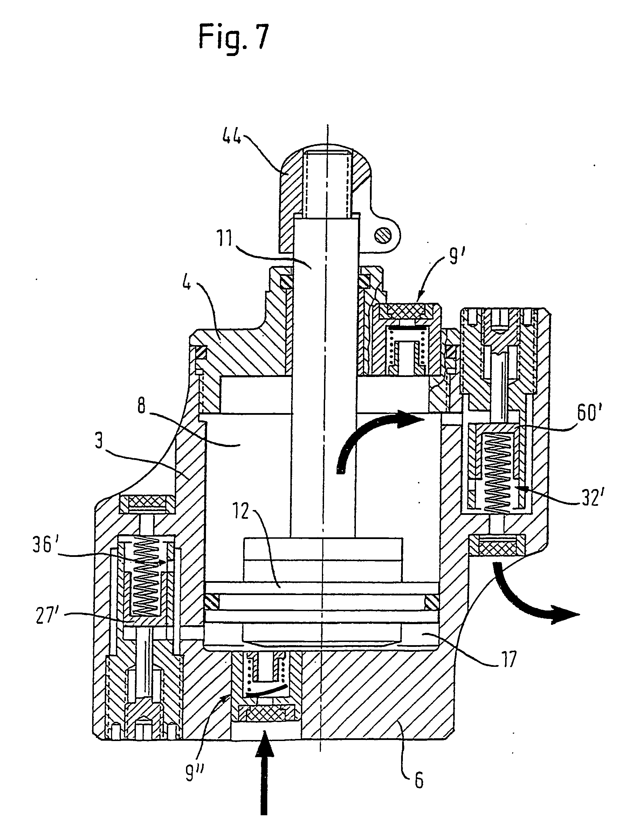

In einer weiteren Ausführungsform ,die in den Fig. 6 und 7 gezeigt ist, ist keine der Drosseln in dem Kolben 12 vorgesehen, sondern beide Drosseln 32', 36' sind jeweils in einem Durchgang zur Umgebungsluft ausgebildet. In jeder der Kammern 8, 17 ist ein Rückschlagventil 9', 9" vorgesehen, das die jeweilige Kammer 8, 17 mit der Außenseite des Zylinders 3 verbindet und in Richtung der Kammer 8, 17 öffnet.

In der Fig. 6 sind mit Pfeilen schematisch die Strömungswege des Fluids bei einer Flexionsbewegung des künstlichen Kniegelenks dargestellt. Bei der Flexionsbewegung bewegt sich, wie bei der zuvor beschriebenen Ausführungsform, der Kolben 12 nach unten und komprimiert das Fluid in der zweiten Kammer 17. Dabei sperrt das Rückschlagventil 9" an der zweiten Kammer, sodass es für das Fluid keinen Durchgang von der zweiten Kammer 17 zu der Außenseite des Zylinders 3 bildet. Folglich fließt das Fluid von der zweiten Kammer 17 über die Drossel 36' zu der Außenseite des Zylinders 3. Durch das geöffnete Rückschlagventil 9' an der ersten Kammer 8 strömt das Fluid von der Außenseite des Zylinders 3 in die erste Kammer 8. Die Drossel 36' ist wie bei der ersten Ausführungsform mit einem Steuerelement 27' versehen, das die Drossel 36' bei einem hohen Druck in der zweiten Kammer 17 verschließt.

Bei der in Fig. 7 dargestellten Extensionsbewegung bewegt sich der Kolben 12 nach oben und das Fluid in der ersten Kammer 8 wird komprimiert. Durch das Rückschlagventil 9" an der zweiten Kammer 17 strömt Fluid von der Außenseite des Zylinders 3 in die zweite Kammer 17, sodass in dieser kein Unterdruck entsteht. Das Rückschlagventil 9' an der ersten Kammer 8 verhindert ein einfaches Ausströmen des Fluids aus der ersten Kammer 8, sodass dieses über die Drossel 32' von der ersten Kammer 8 zu der Außenseite des Zylinders strömt. Die Drossel 32' an der ersten Kammer 8 ist so wie die Drossel 36' mit einem Steuerelement 60' versehen, das die Drossel 32' bei einem auftretenden hohen Druck in der ersten Kammer 8 verschließt.

Als weitere Alternative ist denkbar, beide Drosseln in dem Kolben 12 oder dem Kolben 12 und der Kolbenstange 11 anzuordnen und die Drosseln jeweils in Reihe mit Rückschlagventilen angeordnet in zwei Kanälen anzuordnen. Mit einer solchen Ausführung ist auch ein geschlossenes System, das ein anderes Fluid als die Umgebungsluft verwendet zu realisieren.

## Patentansprüche

1. Schwungphasensteuervorrichtung für ein künstliches Kniegelenk mit einer Kolbenzylindereinrichtung (2) mit einer ersten Kammer (8) auf der einen Seite des Kolbens (12) und einer zweiten Kammer (17) auf der gegenüberliegenden Seite des Kolbens (12),
und mit einer ersten Drossel (36), die das Austreten eines Fluids aus der ersten Kammer (8) drosselt, und einer ersten Einrichtung (63, 60, 61), die so ausgebildet ist, dass sie bei Erreichen bzw. Überschreiten eines vorbestimmten Drucks in der ersten Kammer (8) die erste Drossel (36) schließt,
und mit einer zweiten Drossel (32), die das Austreten des Fluids aus der zweiten Kammer (17) drosselt, und einer zweiten Einrichtung (27, 30, 51b), die so ausgebildet ist, dass sie bei Erreichen bzw. Überschreiten eines vorbestimmten Drucks in der zweiten Kammer (17) die zweite Drossel (32) schließt.

2. Schwungphasensteuervorrichtung nach Anspruch 1, bei der die erste Drossel (36) in dem Kolben (12, 11) angeordnet ist.

3. Schwungphasensteuervorrichtung nach Anspruch 1 oder 2, bei der die erste Kammer (8) und die zweite Kammer (17) über die erste Drossel (36) verbindbar sind.

4. Schwungphasensteuervorrichtung nach einem der Ansprüche 1 bis 3, bei der die erste Kammer (8) mit einem Fluidreservoir verbindbar ist.

5. Schwungphasensteuervorrichtung nach einem der Ansprüche 1 bis 4, bei der die zweite Drossel (32) auf der dem Kolben (12) abgewandten Ausgangsseite der zweiten Kammer angeordnet ist.

6. Schwungphasensteuervorrichtung nach einem der Ansprüche 1 bis 5, bei der die zweite Kammer (17) über die zweite Drossel (32) mit einem Fluidreservoir verbindbar ist.

7. Schwungphasensteuervorrichtung nach einem der Ansprüche 1 bis 6, bei der das Fluid Luft ist.

8. Schwungphasensteuervorrichtung nach Anspruch 7, bei der das Fluidreservoir aus der Umgebungsluft gebildet ist.

9. Schwungphasensteuervorrichtung nach einem der Ansprüche 1 bis 8, bei der das Fluid ein Hydraulikmedium oder ein Pneumatikmedium ist und das Fluidreservoir mit der Umgebung in Druckausgleich steht.

10. Schwungphasensteuervorrichtung nach einem der Ansprüche 1 bis 9, bei der die erste Einrichtung (60) und/oder die zweite Einrichtung (27) als ein Steuerkolben (60, 27) ausgebildet sind, der entlang eines Weges hin und her bewegbar ist.

11. Schwungphasensteuervorrichtung nach einem der Ansprüche 1 bis 10, bei der die erste Einrichtung (60) und/oder die zweite Einrichtung (27) mit einem eine Rückstellkraft aufbringenden Element (61, 30) versehen sind, das bei Unterschreiten des vorbestimmten Drucks die erste bzw. zweite Drossel (36, 32) wieder freigibt.

## Claims

1. Swing phase control device for an artificial knee joint with a piston-cylinder device (2) with a first chamber (8) on one side of the piston (12) and a second chamber (17) on the opposite side of the piston (12);
and with a first throttle (36) throttling discharge of a fluid from the first chamber (8) and a first device (63, 60, 61) constructed to close the first throttle (36) when a predetermined pressure is reached or exceeded in the first chamber (8) ;
and with a second throttle (32) throttling discharge of the fluid from the second chamber (17) and a second device (27, 30, 51b) constructed to close the second throttle (32) when a predetermined pressure is reached or exceeded in the second chamber (17).

2. Swing phase control device according to claim 1, wherein the first throttle (36) is arranged in the piston (12, 11).

3. Swing phase control device according to claim 1 or 2, wherein the first chamber (8) and the second chamber (17) are connectable via the first throttle (36).

4. Swing phase control device according to one of claims 1 to 3, wherein the first chamber (8) is connectable to a fluid reservoir.

5. Swing phase control device according to one of claims 1 to 4, wherein the second throttle (32) is arranged on the output side facing away from the piston (12) of the second chamber.

6. Swing phase control device according to one of claims 1 to 5, wherein the second chamber (17) is connectable to a fluid reservoir via the second throttle (32).

7. Swing phase control device according to one of claims 1 to 6, wherein the fluid is air.

8. Swing phase control device according to claim 7, wherein the fluid reservoir is formed by the ambient air.

9. Swing phase control device according to one of claims 1 to 8, wherein the fluid is a hydraulics medium or a pneumatics medium and the fluid reservoir is pressure compensated with the surrounding area.

10. Swing phase control device according to one of claims 1 to 9, wherein the first device (60) and/or the second device (27) is formed as a control piston (60, 27) movable to and fro along a path.

11. Swing phase control device according to one of claims 1 to 10, wherein the first device (60) and/or the second device (27) is provided with an element (61, 30) applying a restoring force which unblocks the first or second throttle (36, 32), respectively, again, when the pressure falls below the predetermined pressure.

## Revendications

1. Dispositif de commande de phases d'oscillation pour une articulation du genou artificielle avec un dispositif de cylindre à piston (2) avec une première chambre (8) d'un côté du piston (12) et une seconde chambre (17) à l'opposé du piston (12),
et avec un premier étrangleur (36), qui réduit l'arrivée d'un fluide provenant de la première chambre (8), et un premier dispositif (63, 60, 61), qui est conçu de telle manière qu'il ferme le premier étrangleur (36) lorsque la pression atteint ou dépasse une valeur pré-réglée dans la première chambre (8),
et avec un second étrangleur (32), qui réduit l'arrivée d'un fluide provenant de la seconde chambre (17), et un second dispositif (27, 30, 51b), qui est conçu de telle manière qu'il ferme le second étrangleur (32) lorsque la pression atteint ou dépasse une valeur pré-réglée dans la seconde chambre (17).

2. Dispositif de commande de phases d'oscillation selon la revendication 1, dans lequel le premier étrangleur (36) est disposé dans le piston (12, 11).

3. Dispositif de commande de phases d'oscillation selon la revendication 1 ou 2, dans lequel la première chambre (8) et la seconde chambre (17) sont raccordables par le premier étrangleur (36).

4. Dispositif de commande de phases d'oscillation selon l'une quelconque des revendications 1 à 3, dans lequel la première chambre (8) est raccordable à un réservoir à fluide.

5. Dispositif de commande de phases d'oscillation selon l'une quelconque des revendications 1 à 4, dans lequel le second étrangleur (32) est disposé à la sortie de la seconde chambre orientée vers le piston (12).

6. Dispositif de commande de phases d'oscillation selon l'une quelconque des revendications 1 à 5, dans lequel la seconde chambre (17) est raccordable par le second étrangleur (32) à un réservoir à fluide.

7. Dispositif de commande de phases d'oscillation selon l'une quelconque des revendications 1 à 6, dans lequel le fluide est de l'air.

8. Dispositif de commande de phases d'oscillation selon la revendication 7, dans lequel le réservoir à fluide est formé à partir de l'air ambiant.

9. Dispositif de commande de phases d'oscillation selon l'une quelconque des revendications 1 à 8, dans lequel le fluide est un médium hydraulique ou pneumatique et dans lequel la pression du réservoir à fluide est compensée par l'environnement.

10. Dispositif de commande de phases d'oscillation selon l'une quelconque des revendications 1 à 9, dans lequel le premier dispositif (60) et/ou le second dispositif (27) sont conçus comme des pistons de commande (60, 27) qui sont mobiles le long de leur course selon un va-et-vient.

11. Dispositif de commande de phases d'oscillation selon l'une quelconque des revendications 1 à 10, dans lequel le premier dispositif (60) et/ou le second dispositif (27) sont équipés d'un élément produisant une force de rappel (61, 30) qui en cas de baisse sous la pression pré-réglée débloque de nouveau le premier et/ou le second étrangleur (36, 32).
